**Europäisches Patentamt**

**European Patent Office**

**Office européen des brevets**

(19)

(11) Veröffentlichungsnummer: **0 150 462**
**B1**

(12) # EUROPÄISCHE PATENTSCHRIFT

(45) Veröffentlichungstag der Patentschrift:
**11.05.88**

(21) Anmeldenummer: **84116014.6**

(22) Anmeldetag: **20.12.84**

(51) Int. Cl.⁴: **A 61 M 25/00, A 61 M 1/34**

(54) Doppellumiger Katheter für eine Vorrichtung zur in-vivo-Reinigung von Blut.

(30) Priorität: **12.01.84 DE 3400874**

(43) Veröffentlichungstag der Anmeldung:
**07.08.85 Patentblatt 85/32**

(45) Bekanntmachung des Hinweises auf die Patenterteilung:
**11.05.88 Patentblatt 88/19**

(84) Benannte Vertragsstaaten:
**BE CH DE FR GB IT LI SE**

(56) Entgegenhaltungen:
**EP - A - 0 025 704**
**EP - A - 0 036 642**
**EP - A - 0 101 890**
**DE - A - 2 703 087**
**DE - A - 2 813 275**

(73) Patentinhaber: **Aigner, Karl, Dr., Uhlandstrasse 5,
D-6301 Pohlheim (DE)**

(72) Erfinder: **Aigner, Karl, Dr., Uhlandstrasse 5,
D-6301 Pohlheim (DE)**

(74) Vertreter: **Sternagel, Hans-Günther, Dr. et al,
Patentanwälte Dr. Michael Hann Dr. H.-G. Sternagel
Sander Aue 30, D-5060 Bergisch Gladbach 2 (DE)**

**Beschreibung**

Gegenstand der Erfindung ist ein verbesserter doppellumiger Katheter und eine Vorrichtung zur in-vivo-Reinigung von Blut mit dem doppellumigen Katheter, mit der aus einer Vene abgepumptes Blut einer intensiven Ultrafiltration unterzogen werden kann und das Retentat zusammen mit einer dem Filtrat etwa volumenmässig entsprechenden Menge Substitutflüssigkeit wieder der Vene zugeführt werden kann. Die Vorrichtung ist insbesondere dazu geeignet, im Rahmen intraarterieller Chemotherapie in hoher Konzentration eingebrachte Stoffe herauszufiltrieren, ehe das venöse Blut ins Herz und anschliessend in den Körperkreislauf gelangt.

Die Ultrafiltration von menschlichem Blut bei Nierenversagen, um dadurch eine Nierenersatzfunktion zu erreichen, ist bekannt. Dabei wird Blut aus einer Arterie entnommen und unter dem Eigendruck durch ein Ultrafilter oder ein sogenanntes Hämofilter geleitet und das Retentat gegebenenfalls mit Substitutflüssigkeit in eine Vene zurückgeführt. Es werden auch Pumpen zwischengeschaltet, wenn Blutentnahme und Rückführung aus einem Gefäss erfolgen sollen.

Um dies zu ermöglichen, wurden zahlreiche doppellumige Katheter entwickelt.

In der DE-A-28 13 275 ist ein doppellumiger Katheter mit koaxialer Anordnung der Katheterrohre beschrieben, bei dem das äussere Katheterrohr kürzer ist als das Innenrohr, und das vordere Ende des Innenrohres das vordere Ende des Aussenrohres um etwa 6 mm überragt. Das Innenrohr weist im Bereich seines vorderen Endes einander gegenüberliegende seitliche Öffnungen auf. Das Aussenrohr weist im Bereich seines vorderen Endes seitliche Öffnungen auf, die in axialem Abstand voneinander angeordnet sind. Diese konstruktive Gestaltung des Katheters ermöglicht das Abziehen und Zuführen von Blut über einen extrakorporalen Kreislauf.

In DE-A-30 10 841 ist ein Doppelkatheter beschrieben, dessen Katheterrohre gegeneinander verschiebbar ausgebildet sind und der für die Venenpunktion zur Hämodialyse verwendet werden kann. Durch die Verschiebbarkeit der Einzelkatheter können die Eintrittsöffnungen bzw. Austrittsöffnungen der Einzelkatheter an ihren Spitzen nach dem Einführen in ein Blutgefäss in einem solchen Abstand voneinander plaziert werden, dass das in das Blutgefäss zurückgeführte Blut nicht in den Bereich der Entnahmestelle gelangt.

Aus der EP-A-0 025 704 ist ein doppellumiger Katheter bekant, dessen Lumen koaxial gegeneinander verschiebbar ausgebildet sind, so dass nur ein Teil in das Blutgefäss eingebracht werden muss. Das innere Lumen kann ausgetauscht werden, ohne das äussere mit grösserem Durchmesser aus dem Blutgefäss herausziehen zu müssen. Die Katheterspitze ist offen, und es sind längs des Katheters noch mehrere seitliche Öffnungen vorhanden, die aufgrund der zwei Lumen gleichzeitig Blutentnahme und Rückführung ermöglichen.

In FR-A-2 297 640 ist ein doppellumiger Katheter beschrieben, bei dem eines der Katheterrohre zu einem Ballon aufgeweitet ist. Der aus einem elastischen Material bestehende Ballon wird durch den Druck der zurückgeführten Flüssigkeit so weit aufgeweitet, dass er an der Gefässwand anliegt und den Durchfluss sperrt. Die vor dem Ballon liegende Entnahmestelle wird dadurch von der hinter dem Ballon liegenden Rückführstelle räumlich exakt getrennt.

Diese konstruktive Ausführung hat den entscheidenden Nachteil, dass das Blutgefäss vollständig abgesperrt ist, so dass ein unerwünschter Stau entsteht und auch bei der Rückführung des Blutes aus dem zweiten Katheterrohr in das Gefäss ein inhomogenes Strömungsprofil entsteht. In der als Stand der Technik nach Art. 54 (3) EPÜ zu berücksichtigenden EP-A-0 101 890 ist ein doppellumiger Katheter beschrieben. Bei diesem Katheter weist das erste Katheterrohr eine geschlossene Spitze und mehrere seitlich in der Nähe der Spitze angeordnete Öffnungen auf. Das zweite Katheterlumen ist im ersten angeordnet und endet mit zwei oder mehr Öffnungen in der Aussenwand des ersten Katheterrohres. Der Abstand zwischen der von der Katheterspitze am weitesten entfernten seitlichen Öffnung des ersten Katheterrohres von der nächstliegenden Öffnung des zweiten Katheterrohres beträgt 40–50 mm. Die Endöffnungen des zweiten Katheterrohres in der Wand des ersten Katheterrohres sind so gross wie der Querschnitt des zweiten Rohres. Bei der Verwendung dieses Katheters in Kombination mit jeweils an die Lumen anschliessenden Schlauchverbindungen, wobei über einen Schlauch das erste Katheterrohr über eine zwischengeschaltete erste Schlauchpumpe mit der Eingangsseite eines Ultrafiltrationsfilters verbunden ist und über einen zweiten Schlauch die Ausgangsseite des Ultrafiltrationsfilters für die Rückführung des Retentats mit dem zweiten Katheterlumen verbunden ist und in die Schlauchleitung hinter dem Filter eine Zuführleitung mit einer dazwischengeschalteten zweiten Schlauchpumpe für Substitutflüssigkeit mündet, in die Schlauchleitung vor dem Filter eine Zuführleitung für Antikoagulantien mündet und die Abgangsleitung für Filtrat aus dem Ultrafiltrationsfilter über ein genau einzustellendes Ventil in einem Messgerät endet, zur in-vivo-Reinigung von Blut haben sich einige Nachteile herausgestellt. Insbesondere handelt es sich um fertigungstechnische Schwierigkeiten der Anordnung der Katheterrohre und der zuverlässigen Einfügung des Endes des inneren Lumens in Öffnungen der Aussenwand des zweiten aussenliegenden Lumens. Weiterhin hat sich herausgestellt, dass die Einströmbedingungen durch die seitlichen Öffnungen in der Nähe der Katheterspitze bei Einführung des Katheters in die Vena cava in unmittelbare Nähe des Herzens noch weiter verbesserungsbedürftig sind, wenn im Rahmen intraarterieller Chemotherapie, insbesondere bei der Tumorbehandlung, die geeigneten Chemotherapeutika in möglichst hoher Konzentration an die gewünschte Behandlungsstelle herangebracht werden und diese nach der Behandlungsstelle aus dem venösen Blut der Vena cava wieder herausfiltriert werden müssen, um toxische Wirkungen und Nebenreaktionen hinter der Behandlungsstelle zu vermeiden.

Aufgabe der Erfindung ist die weitere Verbesserung von dem doppellumigen Katheter, um Strömungsstörungen innerhalb des Gefässes sowohl im Bereich der Entnahme von Blut als auch der Rückführung weitgehend zu minimieren. Diese Aufgabe wird durch den doppellumigen Katheter gemäss Anspruch 1 gelöst.

Die Erfindung schliesst auch eine Vorrichtung zur in-vivo-Reinigung von Blut ein mit dem verbesserten doppellumigen Katheter in Kombination mit jeweils an die Lumen anschliessenden Schlauchleitungen, wobei die eine Schlauchleitung das Katheteraussenrohr über eine zwischengeschaltete erste Schlauchpumpe mit der Eingangsseite eines Ultrafiltrationsfilters verbindet und die andere Schlauchleitung die Ausgangsseite des Ultrafiltrationsfilters für die Rückführung des Retentats mit dem Katheterinnenrohr verbindet, wobei in die eine Schlauchleitung vor dem Ultrafiltrationsfilter eine Zuführleitung für Antikoagulantien mündet, wobei in die andere Schlauchleitung hinter dem Ultrafiltrationsfilter eine Zuführleitung mit einer dazwischengeschalteten zweiten Schlauchpumpe für Substitutflüssigkeit mündet, und wobei die Abgangsleitung für Filtrat aus dem Ultrafiltrationsfilter über ein genau einzustellendes Ventil in einem Messgerät endet.

Vorzugsweise ist das Innenrohr 6 cm länger als das Aussenrohr, und die paarweise angeordneten einander gegenüberliegenden Bohrungen sind auf einem etwa 3 cm langen Endstück des Katheterinnenrohres angeordnet. Das äussere Lumen oder äussere Katheterrohr umhüllt das Innenrohr, wobei ein gleitender äusserer glatter Übergang vom Innenrohr auf das Aussenrohr ausgebildet ist. Die jeweils paarweise angeordneten Öffnungen im Aussenrohr sind vorzugsweise auf einer Strecke von 4 cm einander gegenüberliegend angeordnet.

Grundsätzlich können sowohl die seitlichen Ansaugöffnungen im Aussenrohr als auch die seitlichen Rückführöffnungen im Innenrohr jeweils paarweise gegeneinander versetzt angeordnet sein, um ein noch gleichmässigeres Strömungsprofil im Bereich der Ansaug- und der Rückführstelle zu erreichen.

Um die Lage des Katheters in dem Gefäss kontrollieren zu können, ist am Übergang zwischen dem Aussenrohr und dem Innenrohr ein sogenannter Kontrastring angeordnet aus einem Material, das unter Röntgenbestrahlung einen Kontrast mit der Umgebung ergibt, so dass die Stellung des Katheters im eingeführten Zustand erkennbar ist. Geeignete röntgenkontrastgebende Materialien sind edle Metalle, ganz besonders bevorzugt ist Gold.

Der Katheter ist als Doppelschlauch ausgebildet, wobei das äussere Katheterrohr als Absaugschlauch dient und das innenliegende Katheterrohr als Rückführschlauch. Der Querschnitt der beiden Katheterteile kann gleichgross oder unterschiedlich ausgebildet sein. Bevorzugt ist jedoch ein gleichgrosser Querschnitt. Wenn der Katheter von der Leiste aus in der Hauptkörpervene hochgeschoben ist, bis die Katheterspitze vor oder im rechten Vorhof des Herzens liegt, hat es sich zur Vermeidung strömungsbedingter Störungen, die zu Herz-Rhythmus-Störung führen können, als besonders günstig erwiesen, die Katheterspitze offen auszubilden und zusätzlich 4 Paare von seitlichen Öffnungen in bestimmtem Abstand voneinander anzuordnen. Als besonders geeignet hat sich ein Abstand von 8–12 mm erwiesen. Ganz besonders bevorzugt ist ein Abstand der Paare voneinander von jeweils 10 mm. Eine gleichartige Anordnung von paarweisen Öffnungen hat sich auch für die Ansaugöffnungen im Aussenrohr als besonders günstig erwiesen. Um einen ausreichenden Abstand zwischen Entnahme und Rückführstelle in dem Gefäss zu gewährleisten, ist das innere Katheterrohr an der Spitze des Katheters 4–6 cm länger ausgebildet als das Aussenrohr. Die Gesamtlänge des Katheters beträgt 65–70 cm, vorzugsweise 68 cm, wobei das Aussenrohr eine Länge von 62 cm aufweist, d.h. dass auch das Innenrohr am hinteren Ende des Katheters länger ist als das Aussenrohr. Der Querschnitt der Katheterrohre und die Öffnungen für den Austritt und die Wiedereinführung sind so ausgebildet, dass eine Förderleistung von mindestens 600 ml pro Minute ohne grösseren Druckabfall bewältigt werden kann. Die seitlichen Öffnungen in Kombination mit einer Öffnung an der Spitze des Katheters verhindern das Auftreten einer zu grossen Pressstrahlwirkung bei derartigen Förderleistungen.

Um einen sicheren Anschluss am hinteren Ende des Katheters zu gewährleisten, ist dort ein Zweiwegeanschlussstück angeordnet. An diesem sind die Enden der Katheterrohre in einem eine Hülse aufweisenden hülsenförmigen Anschlussstück, dessen Innenbohrung grösser ist als der Aussendurchmesser des Katheterinnenrohres, so dass zwischen dem in die Hülse eingeführten Katheterinnenrohr und der Bohrungswand ein Ringspalt ausgebildet ist, in Längsrichtung gegeneinander versetzt fest eingespannt. Der Ringspalt steht in Verbindung mit der Innenbohrung eines seitlich abgeführten Anschlussstutzens. Der seitliche Anschlussstutzen kann im rechten Winkel, aber auch schräg abgeführt sein, so dass ein ypsilonförmiges Anschlussstück entsteht.

Um das Einklemmen der Katheterrohre zu ermöglichen, ist das vordere Ende des Anschlussstückes konisch zulaufend ausgebildet, und der Aussenschlauch wird mittels einer Überwurfmutter, die auf ein Aussengewinde auf der Hülse aufschraubbar ist, eingespannt. Anstelle eines Gewindes kann auch ein Überwurfkonus zum Einspannen verwendet werden, wobei die feste Verbindung zwischen dem Konus der Hülse, dem Aussenrohr des Katheters und dem Überwurfkonus durch Verkleben hergestellt wird. Das Katheterinnenrohr wird mittels eines in ein Innengewinde am hinteren Hülsenende einschraubbaren Konus, der eine durchgehende Bohrung und ein Aussengewinde aufweist, eingespannt. Auch in diesem Falle ist es möglich, auf eine Gewindeverbindung zu verzichten und den Konus mittels Klebstoffs zu befestigen. Schraubverbindungen sind dann bevorzugt, wenn das Anschlusteil aus Metall ausgebildet ist. Das Zweiweganschlussstück kann jedoch auch aus Kunststoff gefertigt sein. In diesem Falle sind Klebverbindungen anstelle von Gewinden besser geeignet. Jedoch können auch bei Kunststoffteilen entsprechende Innen- und Aussengewinde vorgesehen werden.

Im Falle von Kunststoffen werden thermoplastische, durch Spritzen oder Giessen verformbare Materialien verwendet, die ausserdem noch den Anforderungen an Medizintechnik entsprechen und sich inert gegenüber Körperflüssigkeit verhalten, aber auch sterilisierbar sind.

Der Katheter wird insbesondere in Kombination mit Zusatzteilen zur intraarteriellen Chemotherapie verwendet. Dabei wird das venöse Blut mit einer Schlauchpumpe oder sogenannten Rollerpumpe abgesaugt und mit dem erhöhten Pumpendruck durch ein übliches Ultrafiltrationsfilter gepresst und das Retentat in einer Menge von 400–700 ml pro Minute dem Innenrohr wieder zugeführt. Da bei der Filtration gleichzeitig 100–200 ml pro Minute Filtratflüssigkeit aus dem Ultrafiltrationsfilter austreten, ist die Zuführung einer entsprechenden Menge an Substitutflüssigkeit zum Ausgleich erforderlich. Um dies zu ermöglichen, weist die Schlauchleitung zwischen dem Ultrafiltrationsfilterausgang für das Retentat und dem Katheterinnenrohr einen Anschluss für Substitutflüssigkeit auf, wobei in der Anschluss leitung eine zweite Schlauchpumpe angeordnet ist, um die benötigte Menge an Substitutflüssigkeit in das System einspeisen zu können. Der Filtratabgang wird über ein genau einstellbares Ventil geregelt, und die Abführleitung endet in einem Auffanggefäss mit Messmöglichkeit, um die dem Kreislauf entzogene Flüssigkeitsmenge volumenmässig zu bestimmen.

Zwischen der saugenden Schlauchpumpe und dem Katheteraussenrohr mündet in der Schlauchleitung eine Zuführleitung für Antikoagulantien, z.B. Heparin. Diese etwa 1 m lange Zuführleitung weist einen Innendurchmesser von 1,5 mm und einen Aussendurchmesser von 3 mm auf und ist an ihrem anderen Ende an einen genau einstellbaren Injektionsautomaten angeschlossen.

Das Verhältnis der freien Querschnitte der beiden Katheterrohre beträgt 1:1 bis 2:1, vorzugsweise jedoch 1:1. Der Innendurchmesser des inneren Katheterrohres liegt nicht unter 3 mm und beträgt vorzugsweise 4 mm, so dass sich im Bereich der Spitze ein Aussendurchmesser von etwa 3,5–5 mm ergibt. Das Aussenrohr ist geringfügig grösser, um einen Ringspalt zwischen Innenrohr und Aussenrohr auszubilden.

Die Verbindung vom Katheter zur Schlauchpumpe bzw. Ultrafiltrationsfilter erfolgt über Schlauchleitungen, die an das Zweiweganschlussstück am hinteren Ende des Katheters angeschlossen werden. Vor und hinter der ersten Schlauchpumpe, die das Blut ansaugt und in das Filter drückt, ist jeweils eine eingefasste Kautschukmembran in die Schlauchleitung eingesetzt, um Injektionen oder Entnahme von Kontrollproben mit Injektionsspritzen zu ermöglichen. Weitere solche Membranen können in die Filtratleitung nach dem Ultrafiltrationsfilter vor dem Ventil und in die Schlauchleitung für das Retentat zur Rückführung zum Katheter vorhanden sein. Um den Druck und die Füllung im System besser kontrollieren zu können, kann zwischen der Schlauchpumpe und dem Ultrafiltrationsfilter ein Schlauchstück aus elastischem Material in Kissenform angeordnet sein, das sich unter dem von der Schlauchpumpe erzeugten Druck aufweitet und dessen Volumenvergrösserung oder Verkleinerung ein Mass für den durch die Pumpe erzeugten Druck und den Füllungszustand im System ist.

Von besonderem Vorteil für die Beobachtung des Füllungszustandes ist es, wenn in der Schlauchleitung für das Retentat zum Katheter ein weiteres solches Schlauchstück in Kissenform angeordnet ist. Die Kissen wirken auch als Luftblasenfalle.

Um Wärmeverluste durch Abkühlung innerhalb der Gesamtvorrichtung zur in-vivo-Reinigung von Blut ausgleichen zu können, ist bei einer Ausführungsform in der Rückführschlauchleitung eine etwa 3–7 m, vorzugsweise 5 m lange Schlauchspirale vorhanden, die in einem auf 40 °C temperierten Wasserbad angeordnet ist. Dies ermöglicht eine Aufwärmung des filtrierten Blutes und der zugeführten Substitutflüssigkeit auf die gewünschte Körpertemperatur.

Der Katheter kann aus den für Kathetermaterial üblichen und geeigneten Materialien hergestellt werden. Derartige Materialien verhalten sich neutral gegenüber der Körperflüssigkeit, lassen sich ohne Probleme sterilisieren und sind ausreichend elastisch, aber andererseits auch genügend steif und fest, um in Blutgefässe eingeführt zu werden. Geeignete Materialien sind Polyolefine, polyfluorierte Kohlenwasserstoffpolymere, synthetische Kautschuke, Polyvinylchlorid und dergleichen. Besonders bevorzugte Materialien für Katheter sind Silikonkautschuk und implantierbares Polyvinylchlorid. Für die Schlauchverbindungen sind grundsätzlich vergleichbare Materialien geeignet, insbesondere werden jedoch Polyolefine, fluorierte Kohlenwasserstoffpolymere oder Polyvinylchlorid für die Schläuche verwendet. Bei den Schlauchpumpen handelt es sich um handelsübliche und bekannte Schlauchpumpen oder sogenannte Rollerpumpen, bei denen die Pumpwirkung durch Zusammendrücken des Schlauches erzeugt wird.

Als Ultrafiltrationsfilter sind übliche Ultrafiltrationsfilter mit üblichen Membranen geeignet, sofern die Filterfläche und die Filterleistung ausreichend ist, um die erforderliche Menge an niedermolekularen Produkten aus dem Blut sicher zu entfernen. Die erforderliche Filterfläche liegt in der Grössenordnung von 1–2,5 m², vorzugsweise 1,4–2,0 m². Ganz besonders bevorzugt ist eine Filterfläche von 2 m². Geeignete Membranen sind solche, die Substanzen bis zu einem Molekulargewicht von etwa 40 000 bis 60 000 durchlassen, gegenüber höher molekularen Stoffen jedoch sperren. In besonders gelagerten Fällen können auch andere Membranen eingesetzt werden, die nur niedermolekulare Produkte bis zu einer Molekülgrösse von etwa 20 000 durchlassen.

Der erfindungsgemässe Katheter ist insbesondere geeignet für eine Vorrichtung zur Verwendung für die Cytostatika-Filtration bei intraarterieller Chemotherapie. Die Gesamtvorrichtung ermöglicht eine systemische Behandlung mit lokal hoher Konzentration, wobei nach der Einwirkung die Cytostatika und die anderen therapeutisch wirksamen Substanzen aus dem Blut mittels des Ultrafilters herausfiltriert werden und damit toxische Nebenwirkungen vermieden werden, die insbesondere dann auftreten können, wenn diese Stoffe mit dem Blut bis zum Herzen und dann in den Körperkreislauf gelangen.

Die Erfindung wird nun anhand der Abbildungen noch näher beschrieben.

Abbildung 1 zeigt einen Längsschnitt durch den erfindungsgemässen Katheter.

Abbildung 2 zeigt einen Querschnitt durch die koaxial angeordneten Katheterrohre.

Abbildung 3 zeigt das Zweiwegeanschlussstück im Längsschnitt.

Abbildung 4 zeigt eine schematische Gesamtübersicht der Vorrichtung, die zusammen mit dem Katheter verwendet wird.

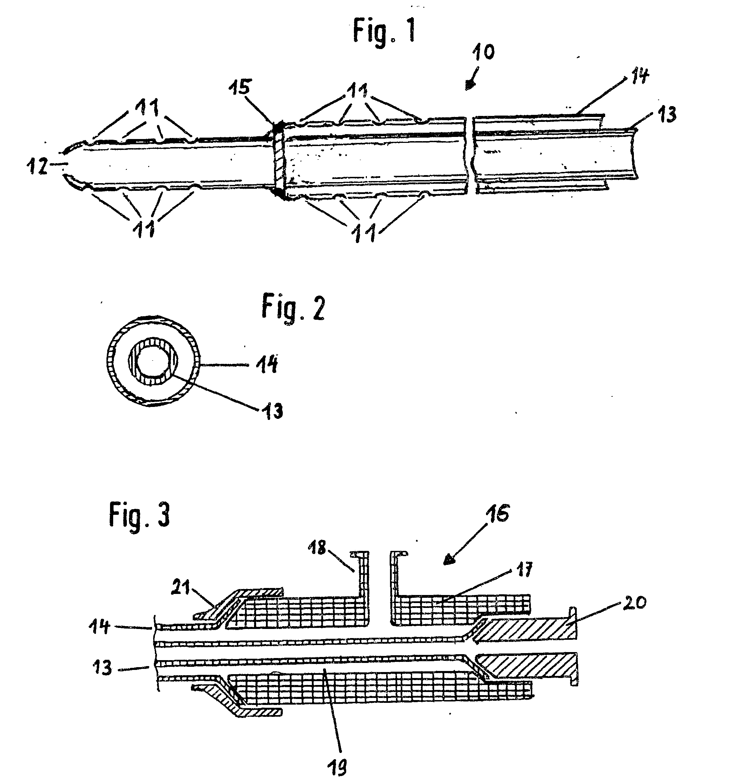

Figur 1 zeigt den doppellumigen Katheter (10) im Längsschnitt mit dem offenen Ende (12) des Innenrohres (13) und den in der Nähe des Endes paarweise angeordneten seitlichen Öffnungen (11). Das äussere Katheterrohr (14) umgibt das Innenrohr (13) auf der Hauptlänge des Katheters und endet etwa 6 cm von der vorderen Spitze des Innenrohres (13) entfernt. An dem konischen Übergang vom Innenrohr (13) auf das Aussenrohr (14), am vorderen Ende des Aussenrohres, ist ein Kontrastring (15) angeordnet. Dieser ermöglicht es, bei Röntgenbestrahlung die Stellung des in die Vene eingeführten Katheters zu erkennen. Auch das Aussenrohr (14) weist in der Nähe seines vorderen Endes 4 paarweise angeordnete Ansaugöffnungen (11) auf. Am hinteren Ende ist das Innenrohr (13) länger ausgebildet als das Aussenrohr (14), um den Anschluss an ein Zweiwegeanschlussstück zu ermöglichen.

Figur 2 zeigt die koaxiale Anordnung von Innenrohr (13) und Aussenrohr (14) auf der Hauptlänge des Katheters.

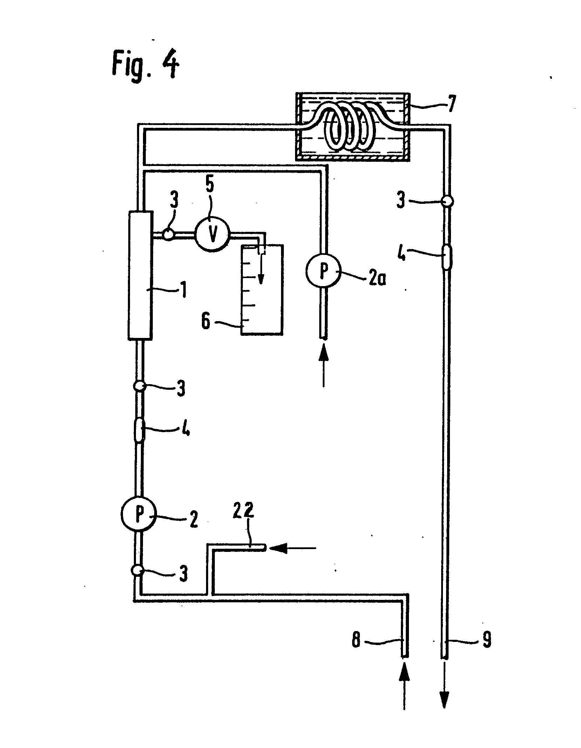

Figur 3 zeigt in schematischer Darstellung einen Längsschnitt durch das Zweiwegeanschlussstück (16). Die Hülse (17) weist eine Innenbohrung (19) auf, die grösser ist als der Aussendurchmesser des Katheterinnenrohres (13) und in etwa mit dem Innendurchmesser des Katheteraussenrohres (14) übereinstimmt. Die Innenbohrung (19) ist am hinteren Ende der Hülse (17) zur Einführung eines konischen Stopfens (20) aufgeweitet. Der Übergang auf den grösseren Durchmesser ist konisch, um das Innenrohr (13) an dieser konischen Fläche mittels der konischen Stirnfläche des Stopfens (20) einspannen zu können. Der Stopfen (20) weist eine durchgehende Innenbohrung auf und am zweiten Ende einen Anschluss für weitere Schlauchverbindungen, vorzugsweise einen Luer-Anschluss. Von der Innenbohrung (19) der Hülse (17) ist seitlich ein Anschlussstutzen (18) abgeführt. In Figur 3 ist dieser rechtwinklig abgeführt, eine schräge Abführung ist jedoch ebenfalls möglich. Auch dieser Stutzen weist am Ende vorzugsweise einen Luer-Anschluss auf, zur Befestigung von Anschlussschläuchen. Das vordere Ende der Hülse (17) ist konisch ausgebildet, um das Aussenrohr (14) des Katheters an dieser Fläche mittels eines Überwurfkonus (21) festspannen zu können. Um den Überwurfkonus (21) und den konischen Stopfen (20) mit der Hülse (17) verbinden zu können, sind entsprechend passende Gewinde, die in der Abbildung 3 nicht wiedergegeben sind, vorhanden, ein Aussengewinde auf der Hülse (17) in der Nähe des vorderen Endes, um eine Überwurfmutter (21) mit Innengewinde aufnehmen zu können, und am Ende der Innenbohrung (19) auf dem Stück mit grösserem Durchmesser ein Innengewinde, um den konischen Stopfen (20) mit einem Aussengewinde einschrauben zu können. Durch entsprechend konische Ausbildungen der Flächen sind Klemmverbindungen möglich, die gegebenenfalls durch Gewinde in der Haltungswirkung verbessert sein können. Um die Klemmverbindungen dauerhaft auszubilden, werden die Katheterrohre zusätzlich mit geeigneten Klebstoffen sowohl innen als auch aussen versehen. Die Verbindung kann auch durch Anspritzen des Anschlussstückes an die Schlauchenden hergestellt werden. Figur 4 zeigt in schematischer Übersicht die Vorrichtung, die in Kombination mit dem Katheter verwendet wird. An die Schlauchleitungen (8, 9) wird der in Figur 1 gezeigte doppellumige Katheter derart angeschlossen, dass das Katheteraussenrohr als Absaugleitung an den Schlauch (8) und das Katheterinnenrohr als Rückführleitung an den Schlauch (9) angeschlossen ist. Über das Katheteraussenrohr wird venöses Blut durch die Schlauchleitung (8) von der Schlauchpumpe (2) angesaugt und durch den anschliessenden Schlauch in das Ultrafiltrationsfilter (1) gedrückt und gelangt von diesem durch die Schlauchleitung (9) in das Katheterinnenrohr. In die Schlauchleitung (8) mündet vor dem Filter (1) die Zuführleitung (22) für Koagulatverhinderungsmittel, die von einer automatischen Injektionsapparatur kommen. In die Schlauchleitung (8) sind vor und hinter der Schlauchpumpe (2) jeweils eingefasste Kautschukmembranen (3) eingeschaltet, um Injektionen vornehmen zu können, bzw. Kontrollproben mit Injektionsspritzen abziehen zu können. Die gleichen Kautschukmembranen sind in der Filtratleitung zwischen dem Filter (1) und dem Ventil (5) und in der Schlauchleitung (9) hinter der im Wasserbad angeordneten Spirale (7) vorhanden.

Mit (4) sind die elastischen Kautschukkissen zur Füllungskontrolle bezeichnet. Vom Ultrafilter (1) geht die Filtratleitung ab, in der das genau einstellbare Ventil (5) angeordnet ist und die in einem Messgefäss (6) zum Auffangen des Filtrats endet. Das Messgefäss weist eine Anzeigeskala auf und hat ein Fassungsvermögen von 1–3 Liter. Hinter dem Ultrafiltrationsfilter (1) befindet sich in der Schlauchleitung (9) die Zuführstelle für Substitutflüssigkeit, die von einer zweiten Schlauchpumpe (2a) eingebracht wird. Die Schlauchpumpe (2a) saugt die Substitutflüssigkeit aus ein oder mehreren, über Schlauchleitung angeschlossenen Vorratsgefässen ausreichender Grösse an. Gegebenenfalls sind in der Schlauchleitung Verzweigungen mittels T-Stücken vorhanden, um mehrere unterschiedliche Substitutflüssigkeiten verwenden zu können. Das Einpumpen der Substitutflüssigkeit, für die vorzugsweise sogenannte Ringer-Lösungen verwendet werden, ist erforderlich, weil sich sonst die grossen Mengen an Substitutflüssigkeit nicht mit der gewünschten Genauigkeit und Geschwindigkeit in das System einbringen lassen. Die Leistung der zweiten Schlauchpumpe (2a) wird auf die durch das einstellbare Ventil (5) aus dem System austretende Flüssigkeitsmenge derart eingeregelt, dass volumenmässig ein Flüssigkeitsverlust möglichst vermieden wird. Grundsätzlich ist es jedoch auch möglich, geringere oder höhere Mengen an Substitutflüssigkeiten einzubringen, wenn dies in einem speziellen Fall im Rahmen der Gesamttherapie erforderlich ist.

Um Abkühlungsverluste auszugleichen, ist vorzugsweise in der Schlauchleitung (9) eine Schlauchspirale (7) angeordnet, die sich in einem Wasserbad befindet, das auf etwa 40 °C temperiert ist. Auf diese Weise können auftretende Wärmeverluste in einfacher Weise kompensiert werden, so dass das Retentat nach der Filtration mit gewünschter Temperatur in die Vene zurückgeführt werden kann. Die Schlauchleitung zwischen dem Katheter und der Schlauchpumpe hat etwa eine Länge von 1,5 m, der Schlauchabstand zwischen Schlauchpumpe und Filter beträgt etwa 1 m, und etwa 2 m Schlauchleitungen ohne die Schlauchspirale sind zum Anschluss des Schlauches (9) an den Katheter erforderlich. Die Schlauchleitungen haben einen Innendurchmesser von etwa 5 mm und einen Aussendurchmesser von etwa 7 mm.

Die Anschlussstellen des Zweiwegeanschlussstückes weisen ebenso wie die Schläuche Farbmarkierungen auf, z.B. rot und blau, um Verwechselungen beim Anschliessen zu vermeiden.

Die Gesamtvorrichtung ist als ein sogenannter Filtrationsset zusammen mit dem Katheter ausgebildet. Vorzugsweise wird der Set als Ganzes in den Verkehr gebracht, wobei eine sterile Verpackung bevorzugt ist, um die Gesamtkombination sofort einsatzfähig zu haben. Grundsätzlich ist jedoch auch eine Auftrennung des Sets in steril und nicht steril verpackte Teile möglich.

## Patentansprüche

1. Doppellumiger Katheter (10) mit koaxialer Anordnung der beiden Katheterrohre, bei dem

(a) das äussere Katheterrohr (14) das Innenrohr (13) auf der Hauptlänge des Katheters (10) umgibt, kürzer ist als das die offene Katheterspitze (12) bildende Innenrohr (13), in Abstand von der Spitze (12) des Innenrohres (13) endet und in der Nähe seines vorderen Endes seitliche Ansaugöffnungen (11) in Abstand voneinander aufweist,

(b) das Innenrohr (13) von der Spitze beginnend Paare von seitlichen Öffnungen (11) aufweist, dadurch gekennzeichnet, dass

(c) das Innenrohr (13) an der Spitze des Katheters (10) 4 bis 6 cm länger ist als das äussere Katheterrohr (14) und die Paare von seitlichen Öffnungen (11) 4 Paare in jeweils einem Abstand zwischen 8 und 12 mm sind,

(d) die seitlichen Ansaugöffnungen (11) am vorderen Ende des äusseren Katheterrohres (14) 4 Paare von seitlichen Öffnungen (11) auf einer Länge von 3,5 bis 6 cm in einem Abstand von jeweils 8 bis 12 mm sind, und

(e) am glatten Übergang vom äusseren Katheterrohr (14) auf das Innenrohr (13) ein Ring (15) aus einem unter Röntgenstrahlung durch Kontrastbildung erkennbaren Material vorhanden ist.

2. Doppellumiger Katheter nach Anspruch 1 mit einem Zweiwegeanschlussstück (16) am hinteren Ende, dadurch gekennzeichnet, dass die hinteren Enden der Katheterrohre (13, 14) in einem eine Hülse (17) aufweisenden hülsenförmigen Anschlussstück (16), dessen Innenbohrung (19) grösser ist als der Aussendurchmesser des Katheterinnenrohres (13), so dass zwischen dem in die Hülse (17) eingeführten Katheterinnenrohr (13) und der Bohrungswand ein Ringspalt ausgebildet ist, in Längsrichtung gegeneinander versetzt fest eingespannt sind und der Ringspalt in Verbindung mit der Innenbohrung eines seitlichen Anschlussstutzens (18) steht.

3. Doppellumiger Katheter nach Anspruch 2, dadurch gekennzeichnet, dass das äussere Katheterrohr (14) am konisch zulaufenden vorderen Ende des eine Hülse (17) aufweisenden Anschlussstückes (16) mittels einer Überwurfmutter (21), die auf ein Aussengewinde auf der Hülse (17) aufschraubbar ist, eingespannt ist, und dass das Katheterinnenrohr (13) mittels eines in ein Innengewinde am hinteren Hülsenende einschraubbaren Konus (20), der eine durchgehende Bohrung und ein Aussengewinde aufweist, eingespannt ist.

4. Vorrichtung zur in-vivo-Reinigung von Blut mit einem doppellumigen Katheter (10) nach Ansprüchen 1 bis 3 in Kombination mit jeweils an die Lumen anschliessenden Schlauchleitungen (8, 9), wobei die eine Schlauchleitung (8) das Katheteraussenrohr (14) über eine zwischengeschaltete erste Schlauchpumpe (2) mit der Eingangsseite eines Ultrafiltrationsfilters (1) verbindet und die andere Schlauchleitung (9) die Ausgangsseite des Ultrafiltrationsfilters (1) für die Rückführung des Retentats mit dem Katheterinnenrohr (13) verbindet, wobei in die eine Schlauchleitung (8) vor dem Ultrafiltrationsfilter (1) eine Zuführleitung (14) für Antikoagulantien mündet, wobei in die andere Schlauchleitung (9) hinter dem Ultrafiltrationsfilter (1) eine Zuführleitung mit einer dazwischengeschalteten zweiten Schlauchpumpe (2a) für Substitutflüssigkeit mündet, und wobei die Abgangsleitung für Filtrat aus dem Ultrafiltrationsfilter (1) über ein genau einzustellendes Ventil (5) in einem Messgerät (6) endet.

**Revendications**

1. Cathéter à deux conduits (10) à agencement coaxial des deux tubes-cathéters, dans lequel:

(a) le tube-cathéter extérieur (14) entoure le tube intérieur (13) sur la plus grande partie de la longueur du cathéter (10), est plus court que le tube intérieur (13) formant la pointe ouverte (12) du cathéter, se termine à une certaine distance de cette pointe (12) du tube intérieur (13) et comporte, au voisinage de son extrémité antérieure, des orifices latéraux d'aspiration (11) espacés entre eux,

(b) le tube intérieur (13) comporte, en commençant à partir de sa pointe, des paires d'orifices latéraux (11), caractérisé en ce que:

(c) le tube intérieur (13) est, à la pointe du cathéter (10), plus long de 4 à 6 cm que le tube-cathéter extérieur (14) et les paires d'orifices latéraux (11) sont au nombre de quatre et espacées deux à deux d'une distance comprise entre 8 et 12 mm,

(d) les orifices latéraux d'aspiration (11) situés à l'extrémité antérieure du tube-cathéter extérieur (14) forment quatre paires d'orifices latéraux (11) disposées sur une longueur d'environ 3,5 à 6 cm et espacées deux à deux d'une distance de 8 à 12 mm, et

(e) à l'endroit de la transition progressive faisant passer du tube-cathéter extérieur (14) au tube intérieur (13), il est prévu un anneau (15) en un matériau repérable par formation de contraste sous exposition aux rayons X.

2. Cathéter à deux conduits suivant la revendication 1, comportant une pièce de raccordement à deux voies (16) à son extrémité postérieure, caractérisé en ce que les extrémités postérieures des tubes-cathéters (13, 14) sont immobilisées par serrage, en pouvant être décalées l'une par rapport à l'autre dans la direction longitudinale, dans une pièce tubulaire creuse de raccordement (16) comprenant un manchon (17) et dont l'alésage intérieur (19) est plus grand que le diamètre extérieur du tube-cathéter intérieur (13), de sorte qu'il se forme un intervalle libre annulaire entre le tube-cathéter intérieur (13) introduit dans le manchon (17) et la paroi de l'alésage, et en ce que cet intervalle libre annulaire communique avec l'alésage intérieur d'une tubulure latérale de raccordement (18).

3. Cathéter à deux conduits suivant la revendication 2, caractérisé en ce que le tube-cathéter extérieur (14) est serré sur l'extrémité antérieure, à profil conique, de la pièce de raccordement (16) comprenant un manchon (17), à l'aide d'un écrou d'accouplement (21) qui peut se visser sur un filetage extérieur de ce manchon (17) et en ce que le tube-cathéter intérieur (13) est serré à l'aide d'un cône (20) qui comporte un alésage le traversant de part en part et un filetage extérieur et qui peut se visser dans un filetage intérieur prévu à l'extrémité postérieure du manchon.

4. Dispositif de purification in vivo du sang comportant un cathéter à deux conduits (10) suivant les revendications 1 à 3 en combinaison avec des tuyauteries souples (8, 9) se raccordant respectivement aux conduits, dans lequel l'une (8) des tuyauteries souples relie le tube-cathéter extérieur (14) au côté entrée d'un filtre d'ultrafiltration (1) en passant par une première pompe tubulaire interposée (2) et l'autre tuyauterie souple (9) relie au tube-cathéter intérieur (13) le côté sortie du filtre d'ultrafiltration (1) qui est prévu pour la réintroduction du rétentat, tandis qu'une tuyauterie (14) d'amenée d'anticoagulants débouche dans la première tuyauterie souple (8) en amont du filtre d'ultrafiltration (1) et que débouche dans l'autre tuyauterie souple (9), en aval de ce filtre d'ultrafiltration (1), une tuyauterie d'amenée de fluide de substitution à laquelle est associée une seconde pompe tubulaire (2a) interposée à cet endroit, la tuyauterie de sortie de filtrat provenant du filtre d'ultrafiltration (1) se terminant, en passant par un robinet (5) réglable de manière précise, dans un appareil de mesure (6).

**Claims**

1. A double lumen catheter (10) having a coaxial arrangement of both catheter tubes, wherein

(a) the outer catheter tube (14) surrounds the inner tube (13) along the main length of the catheter (10), is shorter than the inner tube (13) which forms the open catheter tip (12), terminates a distance from the tip (12) of the inner tube and is provided with lateral suction openings (11) spaced at a distance to each other in the vicinity of its front end,

(b) beginning from the tip, pairs of lateral openings (11) are provided in the inner tube (13), characterized in that,

(c) the inner tube (13) at the tip of the catheter (10) is 4–6 cm longer than the outer catheter tube (14) and the pairs of lateral openings (11) are 4 pairs spaced at distances of between 8 and 12 mm from each other,

(d) the lateral suction openings (11) at the front end of the outer catheter tube (14) are 4 pairs of lateral openings (11) distributed over a length of 3.5–6 cm at intervals of 8–12 mm from each other, and

(e) a ring (15) of a material detectable through contrast formation under X-ray radiation is provided at the smooth transition point from the outer catheter tube (14) to the inner tube (13).

2. The double lumen catheter of claim 1 with a two-way connecting member (16) at its rear end, characterized in that the rear ends of the catheter tubes (13, 14) are firmly clamped at longitudinally staggered distances to each other to a socket (17) of a sleeve-shaped connecting member (16), the inner bore (19) of said connecting member being larger than the outer diameter of the inner catheter tube (13) so that between the inner catheter tube (13) as inserted into the socket (17) and the bore wall an annular gap is provided which communicates with the inner bore of the lateral connecting stud (18).

3. The double lumen catheter of claim 2, characterized in that the outer catheter tube (14) is clamped to the conical front end of the socket (17) of the connecting member (16) by a cap nut (21) screwable to an outer thread at the socket (17), and the inner catheter tube (13) is clamped by a conical member (10) which is designed for screwing into an inner thread at the rear end of the socket, and has a bore therethrough and an outer thread.

4. System for the in-vivo purification of blood with a double lumen catheter (10) according to claims 1 to 3 in combination with the hose lines (8, 9) each connected to the lumena, wherein one of the hose lines connects the outer catheter tube (14) via a first interposed hose pump (2) to the inlet end of an ultrafiltration filter (1) and the other hose line (9 connects the outlet end of the ultrafiltration filter (1) with the inner catheter tube (13 ) for the return of the retentate, where a supply line (14) for anticoagulant agents terminates in the one hose line (8) in front of the ultrafiltration filter, and where a supply line for substitute fluid terminates behind the ultrafiltration filter in the other hose (9) via a second interposed hose pump (2a) and the outflow tube for filtrate from the ultrafiltration filter (1) terminates in a measuring device (6) via an interposed exactly adjustable valve (5).

4/2

## Fig. 1

## Fig. 2

## Fig. 3

2/2

# Fig. 4